# EUROPÄISCHE PATENTSCHRIFT

(11) **EP 0 841 090 B1**
(45) Veröffentlichungstag und Bekanntmachung des Hinweises auf die Patenterteilung: **14.05.2008**
(21) Anmeldenummer: 97119496.4
(22) Anmeldetag: 07.11.1997
(51) Int. Cl.: B01J 23/44, B01J 27/057, C07C 29/56

(54) **Verfahren zur Herstellung von 2-Buten-1-ol-Verbindungen**
Process for production of 2-buten-1-ol compounds
Procédé de production de dérivés du 2-butène-1-ol

(30) Priorität: 12.11.1996 DE 19646679
(43) Veröffentlichungstag der Anmeldung: 13.05.1998
(73) Patentinhaber: BASF SE, 67056 Ludwigshafen (DE)
(72) Erfinder: Bröcker, Franz Josef, Dr., 67061 Ludwigshafen (DE); Aquila, Werner, Dr., 68309 Mannheim (DE); Flick, Klemens, Dr., 76863 Herxheim (DE); Kaibel, Gerd, Dr., 68623 Lampertheim (DE); Langguth, Ernst, Dr., 67281 Kirchheim (DE)
(74) Vertreter: Isenbruck, Günter

(56) Entgegenhaltungen:
- EP-A- 0 647 611
- DE-A- 2 751 766
- FR-A- 2 196 196
- GB-A- 2 053 959

## Beschreibung

Die Erfindung betrifft ein kontinuierliches Verfahren zur Herstellung von 2-Buten-1-ol-Verbindungen.

Aus J. Am. Chem. Soc., 85 (1963), Seiten 1549 bis 1550 ist die Isomerisierung eines ungesättigten Alkohols mit einer Carbonylverbindung eines Metalls der VIII. Gruppe des Periodensystems der Elemente als Katalysator bekannt. Bei diesem Verfahren erhält man zahlreiche Neben- und Folgeprodukte, beispielsweise die entsprechenden Aldehyde.

Aus dem Can. Journ. of Chem., 46 (1968), 2225 bis 2232 ist die thermische Isomerisierung von ungesättigten Alkoholen ohne Katalysator bekannt. Bei den erforderlichen hohen Temperaturen wird eine teilweise Verharzung der Ausgangsverbindungen beobachtet.

In der DE-C-19 01 709 ist ein Verfahren zur Herstellung von Buten-2-ol-4-Verbindungen beschrieben, bei dem Buten-1-ol-4-Verbindungen in Gegenwart von Palladium oder Palladiumverbindungen, wie Palladiumkohle, und Wasserstoff umgesetzt werden.

Bei Verwendung von reinem Palladium in Gegenwart von Wasserstoff wird jedoch in beträchtlichem Maße die Doppelbindung der Verbindungen hydriert und ein gesättigtes Produkt gebildet. Zudem werden niedrigsiedende Verbindungen, wie Kohlenwasserstoffe und Aldehyde als Nebenprodukte gebildet, beispielsweise durch Hydrogenolyse und Isomerisierung. Die Hydrierung der Doppelbindung ist unerwünscht, da bei manchen Butenolen nur geringe Siedepunktdifferenzen zwischen nicht umgesetztem Ausgangsprodukt und Hydrierprodukt bestehen. So beträgt der Siedepunkt von 3-Methyl-3-buten-1-ol beispielsweise bei 1020 mbar 131,5°C, der Siedepunkt des entsprechenden Hydrierproduktes 3-Methyl-1-butanol 130,9°C. Dies macht eine destillative Trennung von Hydrierprodukt und Ausgangsprodukt schwierig.

Aus der DE-C-27 25 965 ist ein Verfahren zur Herstellung eines Alken-2-ol-1 bekannt, bei dem ein Alken-3-ol-1 in einen Borsäurester des Alkenols überführt wird, anschließend die Isomerisierung in Gegenwart eines Palladiumkatalysators, wie Palladium auf Aktivkohle, und Wasserstoff durchgeführt wird und die erhaltene Reaktionsmischung einer Solvolyse unterzogen wird. Durch das Schützen der Hydroxylgruppe des Alken-3-ol-1 durch Veresterung werden weniger Nebenprodukte gebildet. Es sind jedoch zusätzliche Vefahrensschritte notwendig.

Aus der DE-A-27 51 766 ist ein Verfahren zur Isomerisierung von 3-Buten-1-ol-Verbindungen zu den entsprechenden 2-Buten-1-ol-Verbindungen bekannt, wobei die Isomerisierung in Gegenwart von Palladium und Selen oder Tellur als Katalysator und Wasserstoff durchgeführt wird. Als Katalysator wird Palladium und Selen auf Aktivkohle eingesetzt. Als weitere verwendbare Träger sind Bariumsulfat, Kieselgel, Aluminiumoxid und Zeolithe genannt. Die Katalysatoren können auch ohne Träger eingesetzt werden. Es werden relativ hohe Anteile an Leichtsiedern, wie Isopren und Methylbutene gebildet.

EP-A-0 647 611 beschreibt Pd und Te enthaltende Katalysatoren zur Herstellung ungesättigter Glycoldiester.

Die bekannten katalytischen Isomerisierungen werden diskontinuierlich durchgeführt, beispielsweise in einem Rührkessel in Suspensionsfahrweise.

Da die Doppelbindungsisomerisierung von substituierten Butenolen eine Gleichgewichtsreaktion darstellt, werden jedoch keine vollständigen Stoffumsätze erhalten, sondern es bleibt immer ein Teil des Ausgangsstoffes zurück, der für den weiteren Einsatz von gebildeten Nebenprodukten abgetrennt werden muß.

Um die Isomerisierung wirtschaftlicher durchzuführen, sollte die Umsetzung kontinuierlich durchgeführt werden können und zu einem minimalen Anteil an Hydrierprodukten oder Leichtsiedern führen.

Aufgabe der vorliegenden Erfindung ist somit die Bereitstellung eines Verfahrens zur kontinuierlichen Herstellung von 2-Buten-1-ol-Verbindungen durch Isomerisierung von 3-Buten-1-ol-Verbindungen, wobei der Anteil an anfallenden Hydrierprodukten und Leichtsiedern sehr gering ist.

Erfindungsgemäß wird die Aufgabe gelöst durch ein Verfahren zur Herstellung von 2-Buten-1-ol-Verbindungen der allgemeinen Formel (I)

H₂R¹C-R²C=CR³-CR⁴R⁵-OR⁶ (I)

in der die einzelnen Reste R¹, R², R³, R⁴, R⁵ und R⁶ gleich oder verschieden sein können und jeweils ein Wasserstoffatom oder einen aliphatischen Rest, der gegebenenfalls mit OH, OR mit aliphatischem Rest R, Halogen, Carboxyl substituiert sein kann, bedeuten, darüber hinaus R² auch den Rest -CHO bezeichnen kann, R² und R⁵ zusammen mit den zwischen ihnen liegenden Kohlenstoffatomen auch Glieder eines alicyclischen Ringes bedeuten können, und R⁶ auch einen cycloaliphatischen, araliphatischen, aromatischen Rest oder den Rest -C(=O)-R⁷, in dem R⁷ einen aliphatischen, cycloaliphatischen, araliphatischen oder aromatischen Rest bedeutet, bezeichnen kann,
durch Isomerisierung von 3-Buten-1-ol-Verbindungen der allgemeinen Formel (II)

HR¹C=CR²-CHR³-CR⁴R⁵-OR⁶ (II)

in der R¹, R², R³, R⁴, R⁵ und R⁶ die vorgenannte Bedeutung haben, in Gegenwart von Wasserstoff und einem Katalysator, dadurch gekennzeichnet, daß das Verfahren kontinuierlich an einem Festbettkatalysator, der Palladium und Selen auf einem Siliciumdioxidträger enthält und eine BET-Oberfläche von 100 bis 150 m²/g und ein Porenvolumen von 0,8 bis 0,9 cm³/g im Porendurchmesserbereich von 3 nm bis 300 µm aufweist, wobei 85 bis 93 % des Porenvolumens im Porendurchmesserbereich von 10 bis 100 nm liegen und der Katalysator 0,2 bis 0,8 Gew.-% Palladium und 0,02 bis 0,08 Gew.-% Selen, bezogen auf das Gesamtgewicht des Katalysators, enthält, durchgeführt wird.

Vorzugsweise beträgt die BET-Oberfläche 110 bis 130 m²/g. Die BET-Oberfläche wird dabei durch N₂-Adsorption gemäß DIN 66131 bestimmt.

Das Porenvolumen im Porendurchmesserbereich von 3 nm bis 300 µm beträgt 0,8 bis 0,9 cm³/g, insbesondere 0,8 bis 0,85 cm³/g. Dabei liegen 85 bis 93 % dieses Porenvolumens im Porendurchmesserbereich von 10 bis 100 nm. Das Porenvolumen wird dabei durch Hg-Porosimetrie bestimmt.

Vorzugsweise enthält der Katalysator 0,4 bis 0,6 Gew.-% Palladium. Vorzugsweise enthält der Katalysator 0,04 bis 0,06 Gew.-% Selen.

Neben den genannten aktiven Komponenten können weitere Metalle in geringem Umfang auf dem Katalysator vorliegen. Vorzugsweise liegen nur Palladium und Selen auf dem Siliciumdioxidträger vor.

Die erfindungsgemäßen Katalysatoren können nach beliebigen geeigneten Verfahren hergestellt werden. Vorzugsweise werden sie durch Tränken eines Siliciumdioxidträgers mit einer Lösung einer Palladiumverbindung und einer Selenverbindung hergestellt. Dabei können eine oder mehrere Palladiumverbindungen und/oder Selenverbindungen eingesetzt werden. Vorzugsweise werden die Verbindungen in Form von wäßrigen Lösungen eingesetzt. Dabei wird Palladium vorzugsweise in Form von Salzen, wie Palladiumnitrat, oder Komplexen eingesetzt. Selen wird beispielsweise in oxidischer Form eingesetzt. Weitere geeignete Palladium- und Selenverbindungen sind in der DE-A-27 51 766 beschrieben. Dabei kann der Siliciumdioxidträger nacheinander mit Lösungen der einzelnen Verbindungen in beliebiger Reihenfolge getränkt werden, wobei zwischen den einzelnen Tränkungsschritten der Katalysatorträger getrocknet werden kann. Der Katalysatorträger kann jedoch auch mit einer Lösung getränkt werden, die die Verbindungen der aktiven Substanzen in einem entsprechenden gewünschten Verhältnis enthält. Die Konzentration der Lösungen kann so gewählt werden, daß die gewünschte Menge an Palladium und Selen durch einmaliges Tränken auf den Katalysator aufgebracht werden kann. Ein Aufbringen durch mehrmaliges Tränken ist jedoch auch möglich.

Vorzugsweise wird der Katalysatorträger in einer Lösung der aktiven Substanzen bewegt, sodann der getränkte Katalysator bei einer Temperatur von etwa 120°C getrocknet und anschließend bei einer Temperatur von etwa 200°C getempert. Vor oder bei dem Einsatz des Katalysators in der Isomerisierung werden die aktiven Substanzen, d.h. Palladium und Selen in Gegenwart von Wasserstoff reduziert.

Der Katalysatorträger wird vorzugsweise hergestellt, indem Siliciumdioxid aus einer Alkalisilikatlösung ausgefällt und getrocknet und zu Formkörpern gepreßt wird und die so hergestellten Formkörper bei einer Temperatur im Bereich von 400 bis 1100°C, vorzugsweise 600 bis 900°C, insbesondere 800 bis 900°C kalziniert werden.

Dabei wird beispielsweise wäßrige ammoniakalische Alkalisilikatlösung vorgelegt und mit wäßriger Schwefelsäure behandelt, so daß Siliciumdioxid ausfällt. Der erhaltene Niederschlag kann sodann abfiltriert, gewaschen und sprühgetrocknet werden. Dabei wird die Sprühtrocknung vorzugsweise so durchgeführt, daß das gewonnene Silicuimdioxidpulver einen Wassergehalt besitzt, der einem Glühverlust von 25 bis 35 Gew.-% beim Glühen bei 900°C für 2 Stunden entspricht. Das erhaltene Siliciumdioxidpulver kann sodann mit einem Peptisierungsmittel angeteigt und in die gewünschte Form gebracht werden. Bei der Verwendung als Festbettkatalysator kann dieser alle geeigneten makroskopischen Formen aufweisen. Er kann beispielsweise in Form von Strängen, Tabletten, beliebig geformten Pellets, Kugeln oder Ringen eingesetzt werden. Vorzugsweise wird das Siliciumdioxidpulver zu Strängen verpreßt. Die Stränge werden sodann bei 120 bis 150°C getrocknet und anschließend bei 400 bis 1100°C, vorzugsweise 600 bis 900°C, insbesondere 800 bis 900°C kalziniert.

Andere Herstellungsverfahren für den Siliciumdioxidträger können gewählt werden, sofern die erhaltenen Träger die angegebene BET-Oberfläche, Porengröße und Porengrößenverteilung aufweisen.

Die erfindungsgemäße Isomerisierung kann in jeder beliebigen Vorrichtung durchgeführt werden, in der eine kontinuierliche Verfahrensführung möglich ist. Vorzugsweise wird die Isomerisierung in Sumpffahrweise in einem Rohrreaktor durchgeführt, der den Festbettkatalysator enthält. Der Rohrreaktor enthält dabei vorzugsweise im unteren Teil eine Gasverteilung, beispielsweise in Form einer Filterplatte, eines statischen Mischers oder einer Düse. Die Gasverteilung dient zum Zuführen von Wasserstoff, wobei der Reaktorquerschnitt vorzugsweise gleichmäßig begast wird. Die zu isomerisierende 3-Buten-1-ol-Verbindung wird dabei von unten in den Reaktor geleitet und mit Wasserstoff begast. Die Katalysatorbelastung wird dabei so eingestellt, daß am Reaktorausgang ein Umsatz von vorzugsweise 45 bis 65%, besonders bevorzugt 50 bis 60% erzielt wird. Die Wasserstoffbegasung wird in Abhängigkeit von Temperatur und Gesamtdruck so eingestellt, daß ein Wasserstoffpartialdruck von 0,5 bis 5 bar, vorzugsweise 0,5 bis 2 bar, insbesondere 0,6 bis 1 bar aufrechterhalten wird. Der durchgeleitete Wasserstoff kann im Reaktoraustrag nach Auskondensation von Leichtsiedern als Abgas gefahren werden oder wieder in den Prozess zurückgeführt werden.

Die Isomerisierung wird dabei bei Temperaturen zwischen 50 und 120°C, vorzugsweise 80 bis 100°C duchgeführt. Dabei werden je nach eingesetzter Ausgangsverbindung Katalysatorbelastungen von 0,5 bis 5 l/l(Katalysator) x h, vorzugsweise 0,5 bis 1,5 l/l(Katalysator) x h gefahren.

Das aus der Gleichgewichtsreaktion erhaltene Reaktionsproduktgemisch wird vorzugsweise direkt in eine destillative Aufarbeitung geleitet. Dabei wird der nicht umgesetzte Ausgangsstoff isoliert und in die Isomerisierung zurückgeführt. Die destillative Abtrennung des Reaktionsprodukts vom Ausgangsstoff und die Rückführung des Ausgangsstoffes sind für eine wirtschaftliche Durchführung des Isomerisierungsverfahrens notwendig. Die destillative Trennung wird dabei vorzugsweise kontinuierlich in geeigneten Vorrichtungen betrieben.

Das erfindungsgemäße Verfahren kann in Anwesenheit oder in Abwesenheit eines inerten organischen Lösungsmittels durchgeführt werden. Verwendbare inerte organische Lösungsmittel sind beispielsweise Ether, wie Diethylether, Dioxan, Tetrahydrofuran, Alkohole, wie Ethanol, Isobutanol, aromatische oder aliphatische Kohlenwasserstoffe, wie Heptan oder Benzol oder Gemische davon. Vorzugsweise wird ohne inertes organisches Lösungsmittel gearbeitet.

### Ausgangsstoffe

Zur erfindungsgemäßen Herstellung der 2-Buten-1-ol-Verbindungen der allgemeinen Formel (I)

H₂R¹C-R²C=CR³-CR⁴R⁵-OR⁶ (I)

in der die einzelnen Reste R¹, R², R³, R⁴, R⁵ und R⁶ gleich oder verschieden sein können und jeweils ein Wasserstoffatom oder einen aliphatischen Rest, der gegebenenfalls mit OH, OR mit aliphatischem Rest R, Halogen, Carboxyl substituiert sein kann, bedeuten, darüber hinaus R² auch den Rest -CHO bezeichnen kann, R² und R⁵ zusammen mit den zwischen ihnen liegenden Kohlenstoffatomen auch Glieder eines alicyclischen Ringes bedeuten können, und R⁶ auch einen cycloaliphatischen, araliphatischen, aromatischen Rest oder den Rest -C(=O)-R⁷, in dem R⁷ einen aliphatischen, cycloaliphatischen, araliphatischen oder aromatischen Rest bedeutet, bezeichnen kann, werden 3-Buten-1-ol-Verbindungen der allgemeinen Formel (II)

HR¹C=CR²-CHR³-CR⁴R⁵-OR⁶ (II)

eingesetzt, in der R¹, R², R³, R⁴, R⁵ und R⁶ die vorstehende Bedeutung haben.

Vorzugsweise sind die Reste R¹, R², R³, R⁴, R⁵ und R⁶ gleich oder verschieden und jeweils ein Wasserstoffatom oder ein C₁₋₁₂-, vorzugsweise C₁₋₆-Alkylrest, die gegebenenfalls wie vorstehend substituiert sein können. Besonders bevorzugt sind sie Wasserstoffatome oder Methylgruppen.

Gemäß einer Ausführungsform bilden R² und R⁵ zusammen mit den zwischen ihnen liegenden Kohlenstoffatomen Glieder eines 5-, 6- oder 7-gliedrigen, alicyclischen Ringes. R⁶ kann für einen Cycloalkylrest mit 5 bis 7 Kohlenstoffatomen, einen Aralkylrest mit 7 bis 12 Kohlenstoffatomen, einen Phenylrest, Naphthylrest oder den Rest -C(=O)-R⁷ stehen, in dem R⁷ ein C₁₋₁₂-, vorzugsweise C₁₋₆-Alkylrest, ein C₅₋₇-Cycloalkylrest, ein C₇₋₁₂-Aralkylrest, ein Phenylrest oder Naphthylrest ist. Die genannten Reste können auch durch unter den Reaktionsbedingungen inerte Gruppen und/oder Atome, wie Ethergruppen substituiert sein, beispielsweise durch Ethylenoxideinheiten.

Bevorzugte Beispiele erfindungsgemäß verwendbarer Verbindungen der allgemeinen Formel (II) sind 3-Buten-1-ol, 3-Methyl-3-buten-1-ol, 4-Formyl-3-buten-1-ol, 3,2,1-Trimethyl-3-buten-1-ol, 2-Isobutyl-3-buten-1-ol, 3-(2'-Hydroxyethyl)-3-buten-1-ol, 1-Hexyl-3-buten-1-ol, 1-Methylen-2-methylcyclohexan-3-ol, 1-Methylen-2-ethylcyclopentan-3-ol, 1-Methylencyclohexan-3-ol, 1-Methylencycloheptan-3-ol, wie auch die entsprechenden Ethyl-, Cyclohexyl-, Benzyl-, Phenyl- und α-Naphthylether bzw. die entsprechenden Essigsäure-, Cyclohexancarbonsäue-, Benzoesäure-, α-Naphthoesäure- oder Dihydrozimtsäureester.

Besonders bevorzugt werden 3-Methyl-3-buten-1-ol und seine am Sauerstoff substituierten Derivate, wie Acetate oder Ether im erfindungsgemäßen Verfahren eingesetzt.

Die Erfindung wird nachstehend anhand von Beispielen weiter erläutert.

### Beispiel 1

### Katalysatorherstellung

In einem Rührbehälter wird eine wäßrige ammoniakalische Natriumsilikatlösung vorgelegt. Mit wäßriger Schwefelsäure wird unter Rühren Siliciumdioxid ausgefällt. Der erhaltene Niederschlag wird abfiltriert, gewaschen und anschließend sprühgetrocknet. Die Sprühtrocknung wird dabei so durchgeführt, daß das gewonnene Siliciumdioxidpulver einen Wassergehalt besitzt, der einem Glühverlust zwischen 25 und 35 Gew.-% in 2 Stunden bei 900°C entspricht. Das so erhaltene Siliciumdioxidpulver wird mit Wasser und Ammoniak als Peptisierungsmittel angeteigt und zu Strängen mit 3 mm Durchmesser verpreßt. Die Stränge werden in einem Trockenschrank bei 120 bis 150°C getrocknet und anschließend bei 820 bis 870°C kalziniert.

300 g des so gewonnenen Trägermaterials in Form von Strängen mit 3 mm Durchmesser werden in einem Rundkolben an einem Rotationsverdampfer mit einer wäßrigen Lösung aus 13,64 g Palladiumnitratlösung mit 11 Gew.-% Palladium und 0,21 g SeO₂ in 244 g destilliertem Wasser versetzt. Der Kolben wird durch Rotation bei Zimmertemperatur so lange bewegt, bis die gesamte Lösung vom Trägermaterial aufgenommen ist. Anschließend wird der Kolben mit dem Katalysator unter Rotation auf 120°C erhitzt und bei einer Drehgeschwindigkeit von 9 Umdrehungen pro Minute unter Einleitung von 2000 l Luft pro Stunde in 3 Stunden getrocknet. Nach dem Trocknen wird die Temperatur unter ständiger Rotation des Kolbens und Einleitung von 1000 l Luft/h auf 200°C erhöht und der Katalysator für 3 Stunden getempert.

Der so erhaltene Siliciumdioxid Trägerkatalysator enthält 0,5 Gew.-% Palladium und 0,05 Gew.-% Selen, bezogen auf das Gesamtgewicht des Katalysators. Die BET-Oberfläche beträgt 119 m²/g und das Porenvolumen 0,82 cm³/g im Porendurchmesserbereich von 3 nm bis 300 µm. Von diesem Porenvolumen liegen 91,7% im Porendurchmesserbereich von 10 bis 100 nm.

### Beispiel 2

100 ml des nach Beispiel 1 hergestellten Katalysators werden in einem Rohrreaktor mit Doppelmantelheizung und einem Innendurchmesser von 21 mm eingebaut. Aus einem Vorratsbehälter wird mit einer Zulaufpumpe 3-Methyl-3-buten-1-ol von unten in den Reaktor gepumpt. Wasserstoff wird vor dem Reaktoreingang in den Zulauf eindosiert und über eine Glasfritte gleichmäßig über den Reaktorquerschnitt verteilt. Die Isomerisierungstemperatur wird mittels eines Thermostaten über den Reaktordoppelmantel auf 100°C eingestellt. Wasserstoff wird in einer Menge von 2,6 l/h zudosiert. Der Gesamtdruck im Reaktor beträgt 1,1 bar. Bei einer Belastung von 1,0 l/l(Katalysator) x h wird ein Umsatz von 55% und eine Selektivität von 91,5% erzielt.

### Beispiel 3

Das Verfahren aus Beispiel 2 wird wiederholt, jedoch wird bei einer Temperatur von 90°C, einer Katalysatorbelastung von 0,9 1/1(Katalysator) x h und einer Wasserstoffmenge von 3 l/h isomerisiert. Bei einem Umsatz von 75% beträgt die Selektivität 93%.

### Beispiel 4

Das Verfahren aus Beispiel 2 wird wiederholt, jedoch wird bei 80°C, einer Katalysatorbelastung von 0,9 l/l(Katalysator) x h und einer Wasserstoffmenge von 3,8 l/h isomerisiert. Bei einem Umsatz von 55% beträgt die Selektivität 94%.

### Beispiel 5

60 ml des nach Beispiel 1 hergestellten Katalysators werden gemäß dem in Beispiel 2 beschriebenen Verfahren bei 90°C, einer Katalysatorbelastung von 0,72 l/l(Katalysator) x h und einer Wasserstoffmenge von 5 l/h in einem Langzeittest über 1600 h gefahren. Der Umsatz liegt stabil bei 58% mit einer Selektivität von 92%.

Aus den vorstehenden Ergebnissen geht hervor, daß das erfindungsgemäße Verfahren mit hohen Selektivitäten und hohen Umsätzen kontinuierlich durchgeführt werden kann. Auch bei langen Umsetzungszeiträumen bleiben Umsatz und Selektivität stabil. An die kontinuierlich durchgeführte Isomerisierung kann sich eine kontinuierliche destillative Abtrennung des Reaktionsprodukts vom Ausgangsprodukt und die Rückführung des Ausgangsprodukts in die Isomerisierung anschließen, so daß die Isomerisierung insgesamt wirtschaftlich vorteilhaft betrieben werden kann.

## Patentansprüche

1. Verfahren zur Herstellung von 2-Buten-1-ol-Verbindungen der allgemeinen Formel (I)
H₂R¹C-R²C=CR³-CR⁴R⁵-OR⁶ (I)
in der die einzelnen Reste R¹, R², R³, R⁴, R⁵ und R⁶ gleich oder verschieden sein können und jeweils ein Wasserstoffatom oder einen aliphatischen Rest, der gegebenenfalls mit OH, OR mit aliphatischem Rest R, Halogen, Carboxyl substituiert sein kann, bedeuten, darüber hinaus R² auch den Rest -CHO bezeichnen kann, R² und R⁵ zusammen mit den zwischen ihnen liegenden Kohlenstoffatomen auch Glieder eines alicyclischen Ringes bedeuten können, und R⁶ auch einen cycloaliphatischen, araliphatischen, aromatischen Rest oder den Rest -C(=O)-R⁷, in dem R⁷ einen aliphatischen, cycloaliphatischen, araliphatischen oder aromatischen Rest bedeutet, bezeichnen kann,
durch Isomerisierung von 3-Buten-1-ol-Verbindungen der allgemeinen Formel (II)
HR¹C=CR²-CHR³-CR⁴R⁵-OR⁶ (II)
in der R¹, R², R³, R⁴, R⁵ und R⁶ die vorgenannte Bedeutung haben, in Gegenwart von Wasserstoff und einem Katalysator, **dadurch gekennzeichnet, daß** das Verfahren kontinuierlich an einem Festbettkatalysator, der Palladium und Selen auf einem Siliciumdioxidträger enthält und eine BET-Oberfläche von 100 bis 150 m²/g und ein Porenvolumen von 0,8 bis 0,9 cm³/g im Porendurchmesserbereich von 3 nm bis 300 µm aufweist, wobei 85 bis 93 % des Porenvolumens im Porendurchmesserbereich von 10 bis 100 nm liegen und der Katalysator 0,2 bis 0,8 Gew.-% Palladium und 0,02 bis 0,08 Gew.-% Selen, bezogen auf das Gesamtgewicht des Katalysators, enthält, durchgerührt wird.

2. Verfahren nach Anspruch 1, **dadurch gekennzeichnet, daß** das Verfahren in einem Rohrreaktor in Sumpffahrweise durchgeführt wird.

3. Verfahren nach Anspruch 1 oder 2, **dadurch gekennzeichnet, daß** 3-Methyl-3-buten-1-ol eingesetzt wird.

## Claims

1. A process for preparing 2-buten-1-ol compounds of the formula (I)
H₂R¹C-R²C=CR³-CR⁴R⁵-OR⁶ (I)
where the individual radicals R¹, R², R³, R⁴, R⁵ and R⁶ can be identical or different and are each hydrogen or an aliphatic radical which may be optionally substituted by OH, OR where R is aliphatic, halogen or carboxyl, furthermore R² can also be the radical -CHO, R² and R⁵ together with the carbon atoms located between them can also be parts of an alicyclic ring, and R⁶ can also be a cycloaliphatic, araliphatic, aromatic radical or the radical -C(=O)-R⁷, where R⁷ is an aliphatic, cycloaliphatic, araliphatic or aromatic radical,
by isomerizing 3-buten-1-ol compounds of the formula (II)
HR¹C=CR²-CHR³-CR⁴R⁵-OR⁶ (II)
where R¹, R², R³, R⁴, R⁵ and R⁶ are as defined above, in the presence of hydrogen and a catalyst, wherein the process is carried out continuously over a fixed-bed catalyst which comprises palladium and selenium on a silicon dioxide support and has a BET surface area of from 100 to 150 m²/g and a pore volume of from 0.8 to 0.9 cm³/g in the pore diameter range from 3 nm to 300 µm, with from 85 to 93% of the pore volume being in the pore diameter range from 10 to 100 nm and the catalyst comprising from 0.2 to 0.8% by weight of palladium and from 0.02 to 0.08% by weight of selenium, based on the total weight of the catalyst.

2. The process according to claim 1, wherein the process is carried out in the upflow mode in a tube reactor.

3. The process according to claim 1 or 2, wherein 3-methyl-3-buten-1-ol is used.

## Revendications

1. Procédé de préparation de composés de 2-butén-1-ol de formule générale (I)
H₂R¹C-R²-C=CR³-CR⁴R⁵-OR⁶ (I)
dans laquelle les radicaux individuels R¹, R², R³, R⁴, R⁵ et R⁶ peuvent être identiques ou différents et signifient respectivement un atome d'hydrogène ou un radical aliphatique, qui peut être éventuellement substitué avec OH, OR, par un radical aliphatique R, halogène, carboxyle, R² peut par ailleurs désigner également le radical -CHO, R² et R⁵ peuvent signifier, en commun avec les atomes de carbone situés entre eux, également des sommets d'un cycle alicyclique, et R⁶ peut désigner également un radical cycloaliphatique, araliphatique, aromatique ou le radical -C(=O)-R⁷, dans lequel R⁷ signifie un radical aliphatique, cycloaliphatique, araliphatique ou aromatique,
grâce à une isomérisation de composés de 3-butén-1-ol de formule générale (II)
HR¹C=CR²-CHR³-CR⁴R⁵-OR⁶ (II)
dans laquelle R¹, R², R³, R⁴, R⁵ et R⁶ ont la signification mentionnée ci-dessus, en présence d'hydrogène et d'un catalyseur, **caractérisé en ce que** le procédé est mis en oeuvre en continu sur un catalyseur à lit fixe, qui contient du palladium et du sélénium sur un support de dioxyde de silicium et présente une surface BET de 100 à 150 m²/g et un volume de pore de 0,8 à 0,9 cm³/g dans une plage de diamètre de pore de 3 nm à 300 µm, 85 à 93 % du volume de pore se situant dans la plage de diamètre de pore de 10 à 100 nm et le catalyseur contenant 0,2 à 0,8% en poids de palladium et 0,02 à 0,08% en poids de sélénium, par rapport au poids total du catalyseur.

2. Procédé selon la revendication 1, **caractérisé en ce que** le procédé est mis en oeuvre dans un réacteur tubulaire selon un procédé de pied de colonne.

3. Procédé selon la revendication 1 ou 2, **caractérisé en ce que** du 3-méthyl-3-butén-1-ol est utilisé.
